# EUROPEAN PATENT SPECIFICATION

(11) **EP 2 825 626 B1**
(45) Date of publication and mention of the grant of the patent: **11.04.2018**
(21) Application number: 13713105.8
(22) Date of filing: 14.03.2013
(51) Int. Cl.: C12M 3/00, G01N 33/50

(54) **DEVICES AND METHODS FOR OBSERVING EUKARYOTIC CELLS WITHOUT CELL WALL**
VORRICHTUNGEN UND VERFAHREN ZUR BEOBACHTUNG EUKARYOTISCHER ZELLEN OHNE ZELLWAND
DISPOSITIFS ET PROCÉDÉS D'OBSERVATION DE CELLULES EUCARYOTES SANS PAROI CELLULAIRE

(30) Priority: 14.03.2012 EP 12305294
(43) Date of publication of application: 21.01.2015
(73) Proprietor: Centre National de la Recherche Scientifique, 75016 Paris (FR); Université de Strasbourg, 67000 Strasbourg (FR); INSERM (Institut National de la Santé et de la Recherche Médicale), 75013 Paris (FR)
(72) Inventor: RIVELINE, Daniel, F-67000 Strasbourg (FR); WOLLRAB, Viktoria, F-67000 Strasbourg (FR)
(74) Representative: Regimbeau
(86) International application number: PCT/EP2013/055227
(87) International publication number: WO 2013/135809

(56) References cited:
- WO-A1-2010/092116
- US-B1- 6 753 131
- YULI WANG ET AL: "Trapping cells on a stretchable microwell array for single-cell analysis", ANALYTICAL AND BIOANALYTICAL CHEMISTRY, SPRINGER, BERLIN, DE, vol. 402, no. 3, 17 November 2011 (2011-11-17), pages 1065-1072, XP019993327, ISSN: 1618-2650, DOI: 10.1007/S00216-011-5535-9
- S. SHRESTHA ET AL: "PRC1 controls spindle polarization and recruitment of cytokinetic factors during monopolar cytokinesis", MOLECULAR BIOLOGY OF THE CELL, vol. 23, no. 7, 9 February 2012 (2012-02-09), pages 1196-1207, XP055033022, ISSN: 1059-1524, DOI: 10.1091/mbc.E11-12-1008

## Description

### FIELD OF THE INVENTION

The present invention relates to methods and devices for observing the eukaryotic cells and methods for screening compounds of interest. It relates to the field of biology, pharmacology and diagnosis.

### BACKGROUND OF THE INVENTION

The cytokinetic ring is a fundamental structure which causes the separation of cells at the end of mitosis. Cytokinetic ring dysfunction is a typical target for antitumor agents.

Patent application WO 2010/092116 discloses devices allowing the observation of the whole cytokinetic ring in the unique plane of closure by maintaining the ring in the observation plane and methods for using them and preparing them. More particularly, in case of cells without cell wall, this application discloses that the orientation of the cytokinetic ring can be controlled through the cell attachment points into the wells. Indeed, in case of two opposite points of attachment, the cytokinetic ring is perpendicular to the axis joining the two opposite attachment points. Accordingly, for having a cytokinetic ring perpendicular to the support, the two opposite attachment points have to be at the bottom and the upper surfaces of the wells. Obviously, the wells do not include any other substantial attachment points. Therefore, the device further comprises a top covering the wells.

Microfabricated devices with a plurality of wells are known in the prior art.

For instance, Ostuni et al (2001, Langmuir, 17, 2828-2834) discloses arrays of microwells, each well being suitable for attaching one cell. The wells have the following sizes for diameter and depth (in µm): 50 and 1.3; 25 and 5; and 50 and 50, respectively. The surface within the wells is coated with fibronectin.

Yamamura et al (2005, Analytical Chemistry, 77, 8050-8056) discloses a single-cell microarray for B cells and its use for screening. The microchambers are a cylinder having 10-µm width, 12-µm depth and 30-µm pitch. The surface within the wells is rendered hydrophobic by a reactive ion etching.

Ochsner et al (2007, Lab on a Chip, 7, 1074-1077) discloses a device for 3D shape control of single cells. The microwells are coated with fibronectin. The wells' depth is 10 µm and the lateral dimensions were from 81 µm² to 900 µm². The 3D shape is controlled by the form of the wells (square, circle, triangles, rectangles, spindles).

Mi et al (2006, Polymer, 47, 5124-5130) discloses a method of microfabrication of microwells. The microwells can be adapted to contain a single cell.

Wang et al (2012, Anal Bioanal Chem, 402, 1065-1072) discloses the use of an elastic support. For placing cells, the support is stretched, cells are loaded and then the support is very slowly relaxed. The release has to be slow and steady in order to avoid cells expulsion. As a result, cells are really squeezed into the well. Therefore, the trapping mechanism may affect the cell activity since the cells experienced a static mechanism compression. In addition, cells seem to be deformed since, even after removing cells from wells, the released cells are elongated.

However, none is suitable for orientating the closure plane of the cytokinetic ring so as to observe it easily. In conclusion, devices and methods with improved efficiency are still needed and important.

### SUMMARY OF THE INVENTION

In the present invention, it is provided devices and methods for observing the eukaryotic cells without cell wall in a high-throughput way. In particular, devices and methods are suitable and advantageous for observing cytokinetic ring of eukaryotic cells without cell wall, but also to measure expression level or activity of proteins, to determine the localization of proteins or organelles, and to study the structure, shape or integrity of organelles and other cellular elements. First of all, the inventors surprisingly discovered that the two opposite attachment points are not necessary to obtain the appropriate orientation of the cytokinetic ring. They found that the orientation of the cytokinetic ring may be controlled by the shape adopted by cells into the wells. Indeed, wells which constrain the cells to be observed in an oblong shape with the long axis substantially perpendicular to the observation plane (e.g., with a longest axis substantially parallel to the depth of the wells) allow the appropriate orientation of the cytokinetic ring (i.e., with the closure plane in a plane substantially perpendicular to the wells depth and corresponding to the observation plane), without any need to control cell attachments. In addition, the fact that wells constrain cells in an oblong shape allows a reproducible organization of cell conformation rendering possible a high-throughput determination of parameters such as expression level or activity of proteins, the localization and interaction of proteins, the structure, localization, shape or integrity of organelles, cytoskeleton, DNA, or cytokinetic ring. Therefore, a multi-parameters study can be carried out on a high number of cells. In addition, the method of the invention does not lead to deformation of cells (cells are not squeezed). It is fast, easy, and not time-consuming.

Therefore, the present invention relates to a method for observing eukaryotic cells without cell wall, according to claim 1.

In a preferred embodiment, the ratio between the width and the depth of wells is less than 0.8. More preferably, the ratio is between 0.4 to 0.8, more preferably between 0.5 and 0.65.

Preferably, the depth of the wells is less than two diameters of the cells in suspension at a resting phase.

Optionally, the method further comprises a previous step of selecting the suitable device for observing the cells of interest, in particular based on the diameter of the cells in suspension at a resting phase.

Preferably, the step of placing the cells into the wells is carried out by a centrifugation step.

In a preferred embodiment, the step of observing cells comprises observing the cytokinetic ring and the oblong shape of cells orients the closure plane of the cytokinetic ring parallel to an observation, more or less 15°.

Preferably, the step of observing cells comprises one or several of the following steps, optionally for a period of time:
- determining the level of expression of proteins; and/or,
- determining the level of activity of proteins, and/or
- determining the localization or the interaction of proteins; and/or,
- observing the structure, localization, shape and/or integrity of organelles, cytoskeleton, DNA, or cytokinetic ring; and/or
- observing cell apoptosis; and/or
- observing the cytokinetic ring, in particular its closure; and/or
- observing the effect of molecules, antibodies, drugs, or siRNA on the level of expression of proteins, the level of activity of proteins, the localization or interaction of proteins, the structure, localization, shape and/or integrity of organelles, cytoskeleton, DNA, or cytokinetic ring, the closure of the cytokinetic ring.

In a particular embodiment, a combination of these parameters is observed. For instance, at least two, three or four of the above parameters may be combined. In a preferred embodiment, the step of observing cells includes at least observing the cytokinetic ring, in particular its closure.

The present disclosure also relates to a device for observing eukaryotic cells without cell wall, wherein the device comprises a plurality of parallel wells, characterized in that:
- the wells are suitable for containing only one single eukaryotic cell to be observed;
- the ratio between the width and the depth of wells is less than 0.8; and
- the dimensions of the wells constrain the cells to be observed into an oblong shape with a long axis parallel to the depth of the wells.

In a preferred embodiment, the oblong shape of cells orients the closure plane of the cytokinetic ring parallel to an observation, more or less 15°.

In a preferred embodiment of the device, the width of a well is about the diameter of a cell in suspension at a resting phase, more or less 5, 10 or 20 %.

As a top is become useless, the device is not used with a top covering the upper surface of the wells and being coated with molecules promoting cell attachment.

Preferably, the depth of the wells is less than two diameters of the cells in suspension at a resting phase.

In a very particular embodiment of the method or device, the width of the wells is between about 12 to 30 µm, preferably about 20 µm. Preferably, the interior surface of the wells is coated with molecules that promote cell attachment, preferably fibronectin.

Preferably, the device comprises a microfabricated substrate, preferably made of poly(dimethylsiloxane) (PDMS), which may be supported by a plate, preferably of glass, more preferably a glass coverslip. Optionally, the device is not in a stretchable material.

Preferably, the cells are superior eukaryote cells, in particular mammalian cells.

The present invention also relates to the use of a method according to the invention for screening or identifying a molecule of interest, preferably in the pharmaceutical field and/or cosmetics field, in particular a molecule able to modulate the cell division; or for diagnosing a disease, preferably a proliferative disease, in particular a tumor or a cancer; or for assessing the responsiveness and/or the toxicity to a drug.

In a very particular embodiment, the present invention relates to a method for screening or identifying a molecule able to modulate the cell division, in particular a molecule able to modulate the closure of the cytokinetic ring, comprising
- performing the method for observing the cytokinetic ring according to the invention with cells in presence and in absence of a test molecule;
- assessing the closure of the cytokinetic ring of the cells in presence and in absence of the test molecule;
- comparing the closure of the cytokinetic ring of the cells in presence and in absence of the test molecule; and,
- selecting the test molecule for which the closure is significantly different in presence and in absence of the test molecule, thereby identifying a test molecule able to modulate the cell division.

In addition, the present invention relates to a method for *in vitro* diagnosis of a proliferative disorder in a subject comprising:
- performing the method for observing the cytokinetic ring according to the invention with cells of a sample from the subject;
- assessing the closure of the cytokinetic ring of the cells; and,
- comparing the closure of the cytokinetic ring of the cells to the closure of the ring of reference cells.

### BRIEF DESCRIPTION OF THE DRAWINGS

**Figure 1****:** Comparison of the speed of closure for cytokinetic rings on flat surfaces and in egg cups. Note that changes in diameter overlap.
**Figure 2****:** Sequence of pictures of a closing cytokinetic ring at time 0 s, 30 s, 60 s, 120 s. The cytokinetic ring is visualized with the fluorescence from actin (scale bar = 5 µm). Cells were observed in egg cups.
**Figure 3:** Figure 3A) Sequence of pictures for a closing cytokinetic ring at time 0 s, 16.8 s, 33.6 s, 50.4 s, 67.3 s, 84.1 s, 100. 9s after addition of latrunculin A (Lat A, 1.5 µM). The ring is visualized with the fluorescence from actin (scale bar = 5 µm), note that the ring does not close completely. Cells were observed in egg cups. Figure 3B) Ring diameter as a function of time. After addition of latrunculin A, the speed is first slightly increasing, then the ring becomes larger again until the signal vanishes.
**Figure 4:** Figure 4A) Sequence of pictures for a closing ring after time 0 s, 4.5 min, 9 min, 13.5 min and 17.5 min after addition of blebbistatin (100µM) (scale bar = 5 µm). Figure 4B) Ring diameter as a function of time. Blebbistatin slows down and stops the division.
**Figure 5****:** Multiparametric read-outs in egg cups: Figure 5A) Visualization of DNA, a protein (paxillin), and the cytokinetic ring. Figure 5B) Visualization of DNA, the cytokinetic ring and the level of phosphorylation of proteins. The plane of focus is indicated on both panels (scale bar = 5 µm). Figure 5C) Example of quantification of protein concentration: Actin concentration as a function of the radius measured on fixed cells in egg cups.
**Figure 6****:** Egg cups preparation. PDMS is poured on a SU-8 pattern. To obtain the mold, PDMS is then cured and unpeeled. Liquid PDMS is spin coated on a glass coverslip and the mold is placed on the PDMS. After curing, the mold can be unpeeled and the egg cups can be filled with cells.

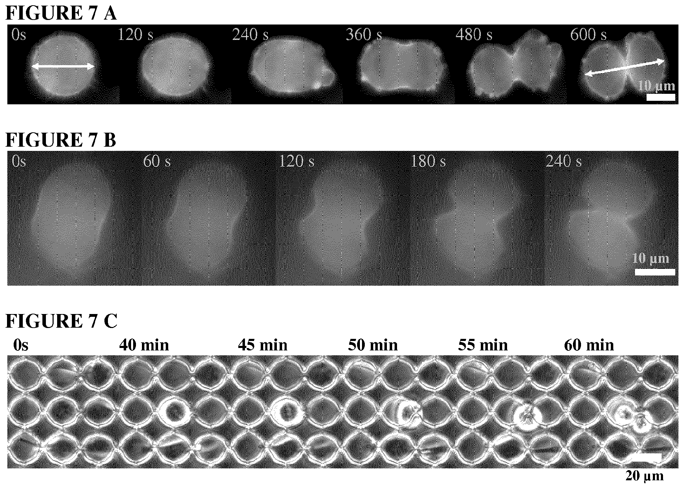

**Figure 7:** Figure 7A) Fluorescent images (actin) of a dividing cell on a glass coverslip. The cell is spherical at the first stage, and then it elongates until it takes a dumbbell shape due to the constriction of the cytokinetic ring in the equatorial plane. We measured the diameter of the round cell and the maximal elongation as indicated by the arrows. (scale bar = 10 µm) Figure 7B) Fluorescent images (actin) of a closing ring after time 0 s, 60 s, 120 s, 180 s and 240 s (scale bar = 10 µm). The dimensions of the egg cups, the width, do not fit the cell (too large, 27 µm diameter). As a consequence, its division axis is tilted. Figure 7C) The width of the wells is too small (20 µm). Cells are pressed out of the well when they are rounding up before the cytokinesis, and the division cannot be observed in the desired plane. The time lapse shows a cell in the well. The cell leaves the well when rounding up and divides outside the cavity (scale bar = 20 µm, phase contrast imaging).

In the examples and figures, it is referred to the wells by the name "egg cups".

### DESCRIPTION OF THE INVENTION

In the present invention, the inventors provide methods for observing the whole cytokinetic ring of cells in the unique plane of closure perpendicular to the observation plane and the devices for using with these methods. More generally, the provided method allows observing the eukaryotic cells without cell wall in a high-throughput way because wells constrain all cells in an oblong and substantially identical shape, leading to a reproducible organization of cell conformation rendering possible a rapid and easy determination with accuracy of parameters such as expression level or activity of proteins, the localization of proteins or organelles, the structure, shape or integrity of organelles, the cytokinetic ring on a high number of cells. The approach is also cheap and allows an automated observation. For instance, in case of cytokinetic rings, more than 1,000 rings can be observed in about one hour. Of course, the observation of the ring can be combined with other parameters of interest. Therefore, the method of the invention is well appropriate for a high-throughput multi-parameters study.

During the cell division, either mitosis or meiosis, the volume of the cells increases, in order to prepare the cell division. Therefore, one can define a first volume of the cell in suspension at the resting phase. The suspension is any fluid compatible with cells viability, such as buffer or culture medium. Cells are rather spherical and a resting diameter may be defined. During the interphase, cells increase in size until the mitosis begins. Therefore, one can define a second volume of the cell in suspension at the end of the interphase or at the beginning of the mitosis. The diameter of the cells at this stage will be referred herein as the mitosis diameter. During the mitosis, cells elongate and adopt an oblong or oval shape. Then, considering the elongated or oblong shape, one can fit an ellipse on the elongated or oblong shape and define a long axis and a short axis. Preferably, one considers the long and short axis of the cell at the cytokinesis, preferably at its beginning.

In order to properly orientate the cell organization and in particular the cytokinetic ring, the inventors unexpectedly observed that the design of the wells can constrain the cells, during the mitosis, to elongate in a direction substantially perpendicular to a plane containing a transversal section of the well or substantially parallel to the depth of the wells. In other words, the long axis of the cells is maintained substantially perpendicular to the support or substantially parallel to the longitudinal axis of the wells. Indeed, in such a configuration, the cytokinetic rings of cells are all substantially contained in a plane perpendicular to the long axis of cells, and then, due to controlled orientation of cells by the wells, substantially parallel to the observation plane.

By "substantially perpendicular" is meant perpendicular, more or less 15°, preferably more or less 10°, more preferably more or less 5°. In other words, the angle is between 75° and 105°, preferably between 80° and 100°, more preferably between 85° and 95°. By "substantially parallel" is meant parallel, more or less 15°, preferably more or less 10°, more preferably more or less 5°. In other words, the angle is between 165° and 195°, preferably between 170° and 190°, more preferably between 175° and 185°. Therefore, the present invention relates to a method for observing eukaryotic cells without cell wall, according to claim 1.

In a preferred embodiment of the method, the ratio between the width and the depth of wells is less than 0.8.

Preferably, the step of observing cells comprises one or several of the following steps:
a) determining the level of expression of proteins; and/or,
b) determining the level of activity of proteins, and/or
c) determining the localization or the interaction of proteins; and/or,
d) observing the structure, localization, shape and/or integrity of organelles, cytoskeleton, DNA, or cytokinetic ring; and/or
e) observing cell apoptosis; and/or
f) observing the cytokinetic ring, in particular its closure; and/or
g) observing the effect of molecules, antibodies, drugs, or siRNA on the level of expression of proteins, the level of activity of proteins, the localization or interaction of proteins, the structure, localization, shape and/or integrity of organelles, cytoskeleton, DNA, or cytokinetic ring, the closure of the cytokinetic ring.

In a preferred embodiment, the step of observing cells comprises observing the cytokinetic ring and the oblong shape of cells orients the closure plane of the cytokinetic ring parallel to an observation, more or less 15°.

Accordingly, the present disclosure relates to a method for observing a cytokinetic ring of eukaryotic cells without cell wall, in particular with its plane of closure perpendicular to the observation plane, comprising
- Providing a device comprising a plurality of wells suitable for containing only one single eukaryotic cell and characterized in that the dimensions of the wells constrain the cells into an oblong shape with a long axis substantially parallel to the depth of the wells;
- Placing the cells into the wells, thereby orienting the closure plane of the cytokinetic ring substantially parallel to an observation plane of the microscope; and
- Observing the cytokinetic ring of the cells.

Optionally, the method further comprises a step selected among the steps a) to e) and g) as disclosed above.

In addition, the present disclosure relates to a device for observing eukaryotic cells without cell wall, wherein the device comprises a plurality of parallel wells, characterized in that:
- the wells are suitable for containing only one single eukaryotic cell to be observed;
- the ratio between the width and the depth of wells is less than 0.8; and
- the dimensions of the wells constrain the cells to be observed into an oblong shape with a long axis substantially parallel to the depth of the wells.

In a preferred embodiment, the device is for observing the cytokinetic ring and the oblong shape of cells orients the closure plane of the cytokinetic ring parallel to an observation, more or less 15°.

More particularly, the wells have a width so as cells either adopt an oblong shape with the long axis substantially parallel to the wells depth (i.e., substantially perpendicular to the support or substantially parallel to the longitudinal axis of the wells) or elongate during mitosis in a direction substantially perpendicular to the support or a plane containing a transversal section of the well (i.e., along a direction substantially parallel to the wells depth or the longitudinal axis of the wells). In other words, the wells have a width so as the cell adopts an oblong shape during the division with the long axis thereof substantially perpendicular to the support. More particularly, the cells have an oblong shape at or during the mitosis.

Accordingly, the device is not used with a top covering the upper surface of the wells and being coated with molecules promoting cell attachment. Therefore, advantageously, the wells are free of upper-top coated with molecules promoting cell attachment. Indeed, extern attachment points become useless for orienting and maintaining the cytokinetic ring in the required position in the wells.

By "perpendicular to the support" is intended to refer to, considering that the support is a flat surface supporting the micro fabricated substrate, a direction perpendicular to that surface of the support. By "parallel to the wells depth" is intended parallel to the longitudinal axis extending from the upper surface of the wells to the bottom of the wells.

By "for containing one single cell" is intended that the wells are suitable for containing one and only one cell.

By "the closure plane of the ring (substantially) parallel to the observation plane" is intended that the closure plane of the cytokinetic ring is (substantially) parallel to the support or a plane corresponding substantially to a plane parallel to the plane in which a transversal section of the well extends. The observation or focal plane is defined herein, where the structure of interest, i.e. the ring, appears entirely on the image. The longitudinal axis of the wells is (substantially) perpendicular to this plane.

By the term "about" is intended to mean the value more or less 5 % thereof.

By "oblong" is intended a shape wherein a long axis is at least 1.1, 1.2, 1.3, 1.4 or 1.5 of the short axis, preferably at least twice.

Considering the feature relating to the dimensions of the wells constrain the cells to be observed into an oblong shape with a long axis substantially parallel to the depth of the wells, the size of the wells can be defined as follow.

One of the main features of the wells in order to properly orientate the cell is the width of the wells. This width may be defined differently depending on the cells to be observed. The width is the parameter selected to constrain the cells to adopt the oblong shape. Preferably, the width of the wells is about the diameter of the cell in a suspension, in particular at a resting stage, more or less 5 or 10%. Optionally, the width of the wells can also be about 50, 60 or 75 % of the diameter of the cell in suspension at the beginning of the mitosis, more preferably about 60, 65, 70 or 75 %. In other words, the width of the wells corresponds about to the short axis of the elongated cell at the mitosis or the cytokinesis in a suspension, optionally with 10, 20, or 30 % more. The lower limit of the wells width depends advantageously on the size of the nucleus. Preferentially, the size of the nucleus is determined at the resting stage of the cells.

The wells width is between about 12 to 30 µm. For instance, a well with a width of 17.5 to 25 µm will be suitable for most of superior eukaryote cells, in particular mammalian cells, for instance for HeLa cells. For instance, the wells width can be about 20 µm. Another feature is the depth of the wells. First of all, there is not a real upper limit if the supply of nutrients for the cells is sufficient. However, in a preferred embodiment, the upper limit of the wells depth is the requirement of having a well suitable for only one single cell. Accordingly, the biggest depth of the wells corresponds to the depth sufficient for containing less than two cells. The shortest depth corresponds to the depth sufficient for containing at the most a single cell. Therefore, the wells can be depth enough to contain a unique cell and to allow it to divide into two cells, but the second cells (i.e., the cell located in the upper part of the wells after division) can be removed by washing the support. Accordingly, the biggest depth of the wells can be for instance 1.5 of the long axis of the elongated cell at the mitosis. Alternatively, the biggest depth of the wells can also be twice the diameter of the resting cell. The shortest depth of the wells is preferably one diameter of the resting cell. The shortest depth of the wells can also be at least 50 % of the long axis. However, preferably, the wells depth is such that the cells are entirely contained into them. Accordingly, in a preferred embodiment, the optimal wells depth is the long axis of the elongated cell at the mitosis.

For instance, a well with a depth of about 30-40 µm will be suitable for most of superior eukaryote cells, in particular mammalian cells, for instance for HeLa cells. In a very particular embodiment, the wells may have a width of 20-25 µm and a depth of about 40 µm.

The well dimensions (i.e., width and depth) can be adapted easily by the man skilled in the art for each specific cell to be studied.

In a preferred embodiment, the ratio between the wells width and the wells depth is less than 1, preferably less than 0.8. Preferably, the ratio is between 0.4 to 0.8, more preferably between 0.5 and 0.65. Preferably, the ratio may be 0.8, 0.75, 0.7, 0.65, 0.6, 0.55 or 0.5 or less.

The wells may have any convenient shape. For instance, the wells may have essentially a cylindrical shape or a prism shape, preferably a right cylindrical shape or a right prism shape. The basis of the prism may be any polygon. In case of a square, the width is a side and not the diagonal. The section of the cylindrical well may be circular. Alternatively, the wells may have a conical or frustoconical shape. The one skilled on the art may easily choose the shape of the wells, the most usual wells having a cylindrical shape. The width of the well is the smallest dimension of the basis. In the context of wells with a cylindrical shape, the width of the wells is the diameter of the circular section of the cylinder, the depth of the wells is the height or length of the cylinder. In case of a square, the width is a side and not the diagonal.

Therefore, the method of the present invention may comprise the step of selecting the suitable device for the cells of interest, in particular based on the cells size, more particularly its diameter (e.g., diameter in suspension at a resting phase) or its nucleus size.

Preferably, the interior surface of the walls of wells is coated with molecules that promote cell attachment. These molecules are well known to those of ordinary skilled in the art and comprise antigens, antibodies, cell adhesion molecules (like cadherins for example), extracellular matrix molecules such as laminin, fibronectin, synthetic peptides, carbohydrates and the like, more preferably fibronectin.

Preferably, the device comprises a micro fabricated substrate advantageously supported by a plate. The microfabricated substrate comprises a high number of wells, e.g., at least or about 10, 20, 50, 100, 150, 200, 300, 400, 500, 1,000, 5,000, 10,000, 50,000, 100,000, 500,000, or 1,000,000 wells. For instance, the microfabricated substrate comprises between 100 and 100,000 wells per cm² of substrate, preferably between 500 and 50,000 wells by cm² of substrate, more preferably between 1,000 and 10,000 wells by cm² of substrate. The wells are sufficiently separated to be discriminated during microscopy observation. In a particular embodiment, the wells are spaced of at least 200 nm, preferably by about 200 nm, 500 nm, 1 µm, 5 µm, or 10 µm. The space between the wells can optionally be treated with a cytophobic material (i.e., preventing cell adhesion), for instance polyethyleneglycol (PEG). The wells of the micro fabricated substrate can have a bottom made of the microfabricated substrate or can go through the microfabricated substrate, the bottom being the support.

By "microfabricated substrate" is intended a microfabricated solid surface including, e.g., silicon, functionalized glass, germanium, ceramic, a semiconductor material, PTFE, carbon, polycarbonate, mica, mylar, plastic, quartz, polystyrene, gallium arsenide, gold, silver, metal, metal alloy, fabric, and combinations thereof. In particular, the microfabricated substrate is made of a solid material preferably biocompatible or with a surface treatment that makes it biocompatible. The following materials can be used for micro fabrication: a polymer that can be crosslinked (PDMS, agar, polyacrylamide (PAA)...), a glassy material (polycarbonate (PC), polystyrene (PS)...), a metal or a semiconductor material. As there is no advantage to use a stretchable material with the method of the invention, it can be chosen to use a material which is not stretchable for preparing the device.

The plate has to be convenient for confocal, optical and/or fluorescence microscopies. An appropriate plate can be any flat substrate promoting a good adhesion with the polymer used for the microstructure (i.e., the microfabricated substrate). In a more preferred embodiment, the plate is a plate of glass, preferably a silanised glass, more preferably a silanised glass coverslip. However, a quartz coverslip is also considered because it allows a high resolution. In a preferred embodiment, the coverslip is as thin as possible. For instance, the thickness of 0.085 to 0.13 mm is convenient.

In a particular embodiment where the objective is placed under the plate, the thickness of the plate and the microfabricated substrate is less than 200 µm, preferably less than 150 µm, more preferably between 100 and 150 µm. However, if the objective is placed up to microfabricated substrate, at the upper surface of the wells, the thickness of the plate and the microfabricated substrate is no more a limitation.

Microfabrication techniques for preparing microfabricated substrates are well-known by the man skilled in the art.

For instance, micro fabrication techniques for preparing the stamp used to produce the microfabricated substrate of the device can be for instance photolithography, thin-film deposition, wet chemical etching, reactive ion etching, inductively coupled plasma deep silicon etching, laser ablation, air abrasion techniques, and other techniques. Polymeric substrate materials are preferred for their ease of manufacture, low cost and disposability, as well as their general inertness. The polymeric substrate materials are preferably cross-linkable. They can be biocompatible and have a weak adhesion for cells. In a preferred embodiment, the substrate material is made of PDMS. Techniques are disclosed for instance in the following patents US 6,753,131; US 6,516,168 and US 6,143,412.

The substrate materials for the stamp can comprise the following polymeric materials without to be limited thereto:
- glassy polymers, such as polystyrene, polymethylmethacrylate (PMMA), polycarbonate, polytetrafluoroethylene (TEFLON®), polyvinylchloride (PVC);
- elastomeric materials polydimethylsiloxane (PDMS), polybutadiene, polyurethane, and the like;
- , gels (agar, PAA...) and the like.

The microfabricated substrates of the device are readily manufactured from masters, using well-known molding techniques, such as injection molding, hot embossing or by molding a polymer that might be crosslinked (soft lithography). The substrate materials for the microfabricated substrates of the device can be the same than stamps and can comprise the following polymeric materials without to be limited thereto:
- glassy polymers, such as polystyrene, polymethylmethacrylate (PMMA), polycarbonate, polytetrafluoroethylene (TEFLON®), polyvinylchloride (PVC);
- elastomeric materials polydimethylsiloxane (PDMS), polybutadiene, polyurethane, and the like;
- , gels (agar, PAA...) and the like, with the advantage of allowing the diffusion of nutrients within the microfabricated structure and therefore the nutrients can diffuse through the side all around the wells.

In a preferred embodiment, the microfabricated substrate is made of biocompatible materials and has a weak adherence for cells. In a most preferred embodiment, the microfabricated substrate is made of PDMS. In a second preferred embodiment, the microfabricated substrate is made of gel including agar, PAA (poly acrylamide) and the like.

In a preferred embodiment, the microfabricated substrate is made of poly(dimethylsiloxane) (PDMS).

Optionally, the device further comprises physical barriers separating several groups of wells from each other on the device. In addition, such a device can further comprise microfluidic system in order to address different samples, culture media or fluids to the groups of wells on the device.

In a particular embodiment, the device may comprise several sets of wells, each set having a distinct size, in particular a different width. In particular, the width can be from µm to mm.

Cells without cell wall are eukaryotic cells, more particularly superior eukaryotic cells such as animal cells, preferably mammalian cells, and more preferably human cells.

Cell can be for example fibroblast, hematopoietic, endothelial and epithelial cell. Preferably, cell is not erythrocyte. Cell can be a stem cell or a somatic cell. In case of stem cells, the cell is preferably a non-human embryonic stem cell. Cells can be primary cells, transdifferentiated cells, dedifferentiated cells, reprogrammed cells, multipotent cells, or pluripotent cells. For instance, cells may be selected non-exhaustively from the group consisting of muscle cells, hematopoietic cells, neural cells, mesenchymal cells, pancreatic cells, hepatic cells, cardiac cells, kidney cells, liver cells, skeletal muscle cells, mammary fatty tissue cells, mammary gland cells, endothelial cells, adipose tissue cells (e.g., adipocyte), thyroid cells, skin cells, prostate cells, lymph node cells, blood cells, retinal cells, dental pulp cells, bladder cells, spleen cells, small intestine cells, colon cells, rectal cells, lung cells, hair follicle cells, intestinal cells, and bone marrow cells. Cell can be derived from a healthy or pathologic tissue or organism. Cells can be from established cell lines or primary cell lines. The cell can be wild type or modified/recombinant cells. In a particular embodiment, the mammalian cell can be a tumor cell, in particular a human tumor cell. Cells can be mononucleated or multinucleated.

Depending on the size of the cells to be observed, a device with the appropriate wells will be selected based on the rules detailed above. For instance, in case of multinucleated cells, the wells width needs to be larger and can reach up to 100 µm, 200 µm, 500 µm or 1 mm.

The cells can be placed into the wells of the device by any means known in the art. For instance, the cells can be introduced in it by centrifugation, opionally followed by washing steps.

The methods of the present invention allow the observation of eukaryotic cells.

In a first embodiment, the method comprises the step of determining the level of expression of proteins. For instance, the level of expression of proteins can be assessed by labeling the protein to be observed or by labeling the mRNA encoding this protein. The methods for labeling proteins or mRNA are well-known in the art. It can be directly labeled by conjugating the protein to a label such as a fluorescent label. Alternatively, it can be indirectly labeled by using antibody specific of the protein to be observed. The detection of mRNA may be carried out by using labeled probes specific of the mRNA to be detected. The level of expression is assessed through the measurement of the intensity of the labeling, preferably the fluorescent or radioactive labeling. The level of expression can be measured at one time or for a period of time. It can be measured for one protein or for several.

In a second embodiment, the method comprises the step of determining the level of activity of a protein. The one skilled in the art can adapt the assay to measure the activity. For instance, in case of kinases or phosphatases, the activity can be assessed by the measurement of the amount of phosphorylated or unphosphorylated proteins which are the substrates of the kinases or phosphatases. For instance, antibodies specific for the phosphorylated or unphosphorylated form of a protein can be used. Other activities can be easily detected such as methylation, sumoylation, and the like. In case of enzymes, the appearance and disappearance of substrates or products can be assessed. In a third embodiment, the method comprises the step of determining the localization or the interaction of proteins. It can be observed by labeling the proteins, preferably by fluorescence, and, in case of the interaction, by using distinct labels for each interaction partners. In case of interaction, a set of fluorophores/quencher or a set of fluorophores allowing fluorescence transfer may be used.

In a fourth embodiment, the method comprises the step of observing the structure, localization, shape and/or integrity of organelles, cytoskeleton, DNA, or cytokinetic ring. The specific case of cytokinetic ring will be discussed in detailed below. The methods for observing organelles, cytoskeleton, DNA, or cytokinetic ring are well known by the one skilled in the art. Organelles may be non-exhaustively selected from endoplasmic reticulum, nucleus, Golgi apparatus, mitochondria, centrioles and vacuole. In a fifth embodiment, the method comprises the step of observing cell apoptosis. The method for observing this phenomenon is well-known in the art. In particular, it can be observed through the DNA fragmentation or the loss of membrane phospholipid asymmetry, or by using luminescent and fluorescent substrates of caspases.

In a sixth embodiment, the method comprises the step of observing the cytokinetic ring of cells. The cytokinetic ring can be observed by microscopy, in particular fluorescence microscopy. Generally, the cytokinetic ring is observed through fluorescent proteins (Glotzer M, Science. 2005 Mar 18;307(5716):1735-9). The fluorescent proteins can be any protein of the cytokinetic ring. In case of fluorescently labeled proteins, the cells are genetically engineered in order to express such fluorescently labeled proteins. Alternatively, the ring can be observed by using fluorescently labeled ring marker or antibody directed against any protein of the ring. For instance, rhodamine or Alexa-phalloidine is suitable for this use, as well as immunofluorescence staining with anti-myosin antibodies (Glotzer M, Science. 2005 Mar 18;307(5716):1735-9).

In an addition embodiment, the method comprises the step of observing the effect of molecules, antibodies, drugs, or siRNA on cells, in particular on the level of expression of proteins, the level of activity of proteins, the localization or interaction of proteins, the structure, localization, shape and/or integrity of organelles, cytoskeleton, DNA, or cytokinetic ring, the closure of the cytokinetic ring. It can be carried at one time or for a period of time. For instance, it can be useful for screening methods, or for methods for determining the efficiency or toxicity of drugs.

In a preferred embodiment of the present invention, a combination of these parameters is observed. Indeed, an advantage of the present method is to allow a multi-parametric analysis of cells. For instance, at least two, three or four of the above parameters may be combined. In addition, the study of these parameters through a period of time allows the analysis of the dynamics. In a preferred embodiment, the step of observing cells includes at least observing the cytokinetic ring, in particular its closure.

In addition to basic research, two other types of application can be envisioned for the methods and device of the invention. The first use is the screening of molecules of interest. Therefore, the present invention relates to the use of the method or device according to the present invention for molecules screening. The second use is the diagnosis or prognosis. Therefore, the present invention concerns the use of the method or device according to the present invention for disease diagnosis, in particular proliferative disease diagnosis, preferably tumor or cancer diagnosis. However, depending on the studied parameter(s), several diseases can be detected such as inflammatory diseases. In addition, the methods are useful for determining the efficiency or toxicity of drugs on cells.

In a very particular embodiment, the present disclosure relates to a method for observing the cytokinetic ring of eukaryotic cells without cell walls, comprising a) providing a device according to the present invention bearing the cells; and b) observing the cytokinetic ring with the closure plane of the cytokinetic ring parallel to the observation plane. In particular, the step a) comprises providing the cells for which the cytokinetic ring has to be observed; selecting a device according to the present invention adapted or suitable for the provided cells (i.e., having wells suitable for constraining cells to be observed into an oblong shape with a long axis parallel to the depth of the wells) and placing the cells on the device. The present disclosure also relates to a method for observing the cytokinetic ring of eukaryotic cells without cell walls, comprising a) providing a device comprising a plurality of wells suitable for containing only one single eukaryotic cell and characterized in that the dimensions of the wells constrain the cells into an oblong shape with a long axis parallel to the depth of the wells; b) placing the cells into the wells, thereby orienting the closure plane of the cytokinetic ring parallel to an observation plane; and c) observing the cytokinetic ring of the cells.

It also concerns the use of a device according to the invention for observing the cytokinetic ring of cells with the closure plane of the cytokinetic ring parallel to the observation plane. More particularly, the step of observing the cytokinetic ring comprises observing the closure of the cytokinetic ring.

In a first embodiment, the step of assessing the closure of the ring comprises the measure of the number of cells having an open ring and of the cells having a closed ring. In a second embodiment, the step of assessing the closure of the ring comprises the measure of the velocity of the ring closure. In a third embodiment, the step of assessing the closure of the ring comprises both the measure of the number of cells having an opened ring and of the cells having a closed ring and the measure of the velocity of the ring closure. In a fourth embodiment, the step of assessing the closure of the ring comprises registering the cytokinetic ring for several cells. By "several" is preferably intended at least 10, 100, 1,000, 10,000, 100,000 or 1,000,000 cells. The cells can be synchronized at the beginning of the method or not.

Optionally, the observation of the cytokinetic ring, in particular to the closure of the cytokinetic ring, can be combined with the observation of other parameters, as detailed above.

It also concerns the use of a device according to the invention for observing the cytokinetic ring of cells with the closure plane of the cytokinetic ring parallel to the observation plane.

In the first use, the device or method of the present invention is used to assay or test the ability of a test molecule to modulate the cell division, in particular to assay or test the ability of a test molecule to modulate the closure of the ring. In particular, a molecule of interest is a molecule able to block or inhibit the cell division, in particular through the blockage or inhibition of the closure of the cytokinetic ring. Accordingly, the present invention concerns a method for screening or identifying a test molecule able to modulate the cell division comprising:
- providing a device according to the present invention bearing cells which have been contacted with a test molecule;
- assessing the closure of the ring of the cells;
- comparing the closure of the ring of the cells in presence and in absence of the test molecule; and,
- selecting the test molecule for which the closure is significantly different in presence and in absence of the test molecule, thereby identifying a test molecule able to modulate the cell division.

Alternatively, the method comprises
- performing the method for observing the cytokinetic ring according to the invention with cells in presence and in absence of a test molecule;
- assessing the closure of the cytokinetic ring of the cells in presence and in absence of the test molecule;
- comparing the closure of the cytokinetic ring of the cells in presence and in absence of the test molecule; and,
- selecting the test molecule for which the closure is significantly different in presence and in absence of the test molecule, thereby identifying a test molecule able to modulate the cell division.

More particularly, the method comprises
- Providing a device comprising a plurality of parallel wells suitable for containing only one single eukaryotic cell and characterized in that the dimensions of the wells constrain the cells into an oblong shape with a long axis parallel to the depth of the wells, more or less 15°, the device bearing cells which have been contacted with a test molecule;
- assessing the closure of the cytokinetic ring of the cells in presence and in absence of the test molecule;
- comparing the closure of the cytokinetic ring of the cells in presence and in absence of the test molecule; and,
- selecting the test molecule for which the closure is significantly different in presence and in absence of the test molecule, thereby identifying a test molecule able to modulate the cell division.

In a particular embodiment, the first step comprises providing cells on which the test molecule has to be tested; selecting a device according to the present invention adapted for the provided cells and placing the cells on the device.

In a first embodiment, the cells are contacting with the test molecule before being placed on the device. Accordingly, the method can comprise previous steps of contacting cells with the test molecule and loading the contacted cells on the device. In an alternative embodiment, the cells are contacting with the test molecule when they are already placed on the device. According, the method can comprise previous steps of loading the cells on the device and contacting cells with the test molecule.

The cells can be synchronized at the beginning of the method or not.

In a first embodiment, the step of assessing the closure of the ring comprises the measure of the number of cells having an open ring and of the cells having a closed ring, and the closure is significantly different in presence and in absence of the test molecule when the number of open and closed ring is significantly different in presence and in absence of the test molecule.

In a second embodiment, the step of assessing the closure of the ring comprises the measure of the velocity of the ring closure, and the closure is significantly different in presence and in absence of the test molecule when the velocity is significantly different in presence and in absence of the test molecule.

In a third embodiment, the step of assessing the closure of the ring comprises both the measure of the number of cells having an opened ring and of the cells having a closed ring and the measure of the velocity of the ring closure.

In a fourth embodiment, the step of assessing the closure of the ring comprises registering the cytokinetic ring for several cells either in presence or in absence of the test molecule. If the test molecule does not modulate the cell division, the ring of cells can statistically have any position. The superposition of the registered rings results in a disk. Alternatively, if the test molecule blocks or inhibits the ring closure, the ring of cells has a higher probability to be opened. The superposition of the registered rings results in a circle. Therefore, a test molecule blocking or inhibiting the ring closure can be selected through the form for the superposition of the registered rings.

The identified molecules have an interest as anti-proliferative agents and the method is a method for screening or identifying a test molecule having anti-proliferative property, in particular anti-tumor property.

The test molecule may be of various origin, nature and composition. It may be any organic or inorganic substance, such as a lipid, peptide, polypeptide, nucleic acid, small molecule, etc., isolated or mixed with other substances. For instance, the test compound can be an antibody, an antisense oligonucleotide, or an RNAi. The molecule may be all or part of a combinatorial library of products, for instance.

The advantage of the device of the present disclosure is that a high number of test molecules can be simultaneously assayed due to the high number of wells and the automated record of the ring closure. In this embodiment, the device according to the present disclosure can comprise several groups of wells on the same plate separated from each other such that each group can be incubated in a different medium. For instance, a group of wells can be contacted with a test molecule and another group can be contacted with another test molecule or without any test molecule. This separation can be provided by a physical barrier such as teflon seal or directly PDMS molded separations. For example, see SPI Teflon® of SPI Supplies, Teflon® Printed Slides of Aname. For instance, each group of wells can comprise at least or about 10, 100, 1,000, 10,000, 100,000 or 1,000,000 wells. Microfluidic system can be further used in order to address a different drug at different locations or groups of wells on the device (Melin J, Quake SR., Annu Rev Biophys Biomol Struct. 2007;36:213-31 ; Hansen C, Quake SR, Curr Opin Struct Biol. 2003 Oct;13(5):538-44 ; Hong JW, Quake SR, Nat Biotechnol. 2003 Oct;21(10):1179-83 ; Quake SR, Scherer A, Science. 2000 Nov 24;290(5496):1536-40). In the second specific use, the method of the present invention is used to diagnose disease, in particular a proliferative disease, preferably cancer or tumor, in a subject. Indeed, the method of the present invention allows the determination of the properties of the ring (e.g., morphology, etc...) as well as the number and the velocity of the cell division.

In the second use, the method of the present invention is used to diagnose proliferative disease, in particular cancer or tumor, in a subject. Indeed, the device or method of the present invention allows the determination of the properties of the ring (e.g., morphology, etc...) as well as the number and the velocity of the cell division. Accordingly, the present invention concerns a method for diagnosing a proliferative disease in a subject or a method for obtaining information useful for diagnosing a proliferative disease in a subject, comprising:
- providing a device according to the present disclosure bearing cells from a subject sample;
- assessing the closure of the ring of the cells; and,
- comparing the closure of the ring of the cells to the closure of the ring of reference cells.

The present invention also concerns a method for diagnosing a proliferative disease in a subject or a method for obtaining information useful for diagnosing a proliferative disease in a subject, comprising:
- performing the method for observing the cytokinetic ring according to the invention with cells of a sample from the subject;
- assessing the closure of the ring of the cells; and,
- comparing the closure of the ring of the cells to the closure of the ring of reference cells.

More particularly, the method comprises
- Providing a device comprising a plurality of parallel wells suitable for containing only one single eukaryotic cell and characterized in that the dimensions of the wells constrains the cells into an oblong shape with a long axis parallel to the depth of the wells, more or less 15°, the device bearing cells of a sample from the subject;
   - assessing the closure of the ring of the cells; and,
   - comparing the closure of the ring of the cells to the closure of the ring of reference cells.

In particular, the first step comprises providing cells from the subject sample to be tested; selecting a device according to the present disclosure adapted for the provided cells and placing the cells on the device.

In a first embodiment, the reference cells are healthy cells (i.e., cells not suffering of proliferative disorder). Accordingly, a significant difference of the ring closure may be indicative of a proliferative disorder or disease. In a second embodiment, the reference cells are cells affected by a proliferative disorder and the absence of a significant difference of the ring closure may be indicative of a proliferative disorder or disease.

In a first embodiment, the step of assessing the closure of the ring comprises the measure of the number of cells having an open ring and of the cells having a closed ring, and the closure is significantly different in presence and in absence of the test molecule when the number of opened and closed ring is significantly different from the reference. In a second embodiment, the step of assessing the closure of the ring comprises the measure of the velocity of the ring closure, and the closure is significantly different from the reference when the velocity is significantly different from the reference. In a third embodiment, the step of assessing the closure of the ring comprises both the measure of the number of cells having an opened ring and of the cells having a closed ring and the measure of the velocity of the ring closure. In a fourth embodiment, the step of assessing the closure of the ring comprises registering the cytokinetic ring for several cells and determining the form of the superposition of the rings.

Further aspects and advantages of the present invention will be disclosed in the following experimental section, which should be regarded as illustrative and not limiting the scope of the present application.

### EXAMPLES

### Cell division is not modified by the new culture condition in egg cups on the device

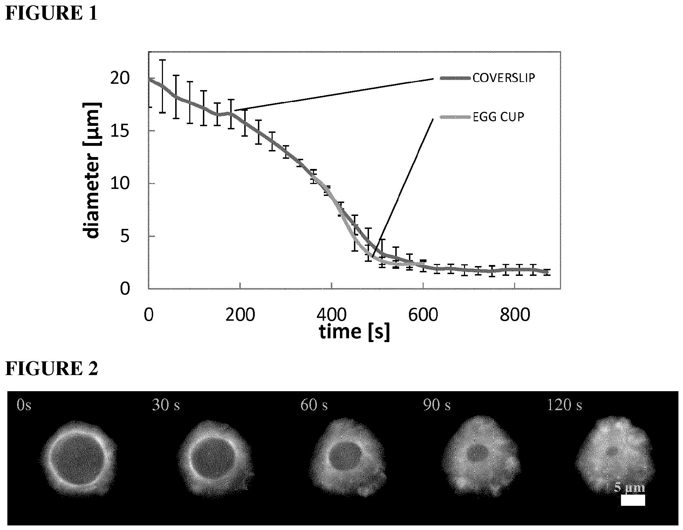

Cell division is unaltered in the micro fabricated substrate in comparison to cells on flat coverslips (see Fig. 1). In both conditions, cells were allowed to grow in the same medium (L-15, 10 % FCS, 2 mM L-Glutamine) at 37 °C. In the device of the invention, cells were centrifuged into cavities (here named *egg cups*). Cells were synchronized before the experiment. In the other configuration, cells were growing on glass coverslips. They were neither synchronized nor centrifuged. The division speeds and temporal behaviors were the same in both conditions (Fig. 1). This shows that the device of the invention does not disturb cell growth.

### Features

Different markers can be used to observe the cytokinetic rings such as GFP-Myosin (Myosin-II heavy chain), GFP-actin or lifeact-mcherry. Lifeact is the name of a small peptide binding filamentous actin (Riedl et al, Nature Methods. 2008, 5(7): 605-607). A sequence of a closing cytokinetic ring is presented in Figure 2.

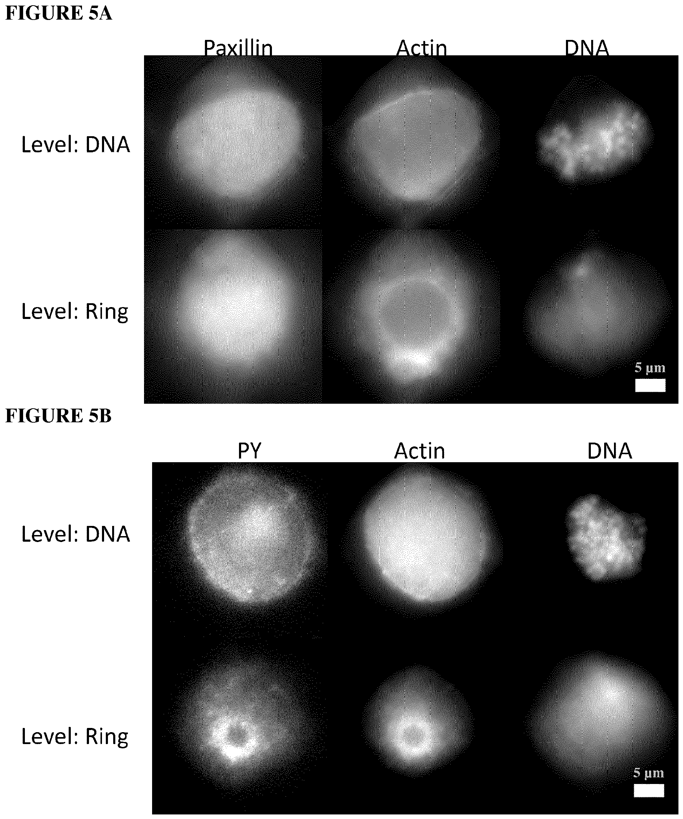

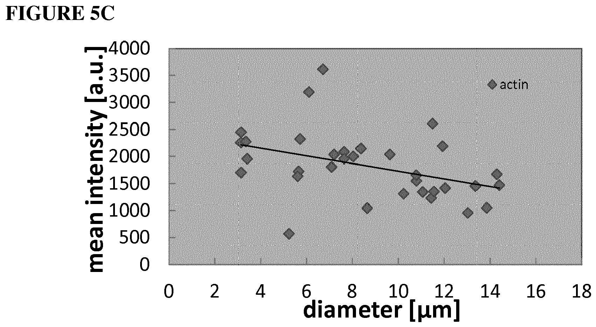

Other features can be revealed with immunofluorescence. Organelles can be visualized, i.e. the ring and the nucleus (see Fig. 5). Levels of proteins expression can also be quantified (see Fig. 5). In addition, activities of proteins can be evaluated such as tyrosine phosphorylation (see Fig. 5). Altogether, the wells (or egg cups) allow multiparametric characterizations of conditions on 'identical' cells with associated perspectives in high content screening.

The dimensions of the egg cups allow an orientation of the cytokinetic ring and other organelles. In the case that the dimensions are not appropriated for the cell (namely, too large), it will be tilted (see Fig. 7 b). For instance, this can be used to differentiate between different cell lines or sizes.

In contrast, if the width of the egg cups is too narrow (smaller than 20µm), cells are submitted to too high constraints during mitotic cell rounding, which results in the expulsion of cells from the well (see Fig. 7 c).

The setup has numerous advantages: (i) The exposure time is 0.6 s or less which allows a high temporal resolution; (ii) During the acquisition, no refocusing is necessary, since the cytokinetic ring remains in the same focal plane; (iii) A high number of cell divisions can be captured in a short time. Since the cell divisions take mainly place in the same focal plane, a high egg cup filling combined with a good synchronization, will give potentially about 200 cell divisions within 10 min; (iv) beyond the ring, any proteins or proteins activity can be quantified quickly with virtually the same cell phenotype repeated in each egg cup, leading to a mean level/activity per condition.

The method is suitable to provide a statistically significant number of cell divisions and activity in an unprecedented time period.

### Drugs are normally altering the closure of the cytokinetic ring on the device.

Actin and myosin are known to be crucial proteins in the closure of the cytokinetic ring (Pollard TD, Curr Opin Cell Biol. 2010, 22(1): 50-56). Different drugs were added, which are interacting with these proteins. An important change in division behavior has been observed.

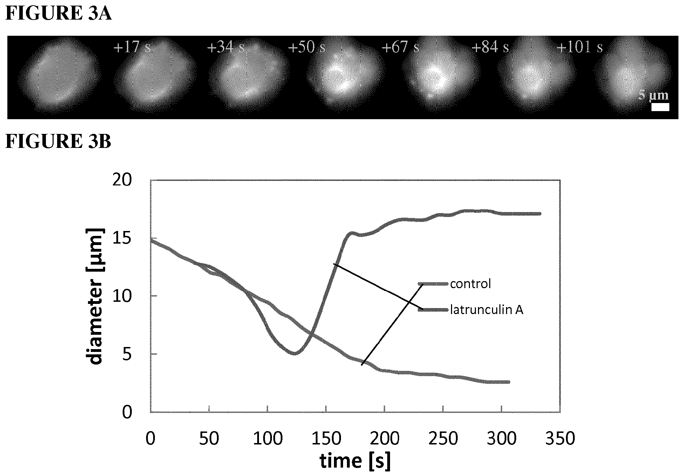

Latrunculin A is a drug which is sequestering monomeric actin (Ayscough K, Methodes Enyzmol. 1998, 298: 18-25). The inventors observed that, after its addition, there was, at first, a slight increase in closing speed, then a slight expansion of the ring. The ring signal lost its intensity until the ring vanished. Cells did not divide in the presence of the drug (see Fig. 3).

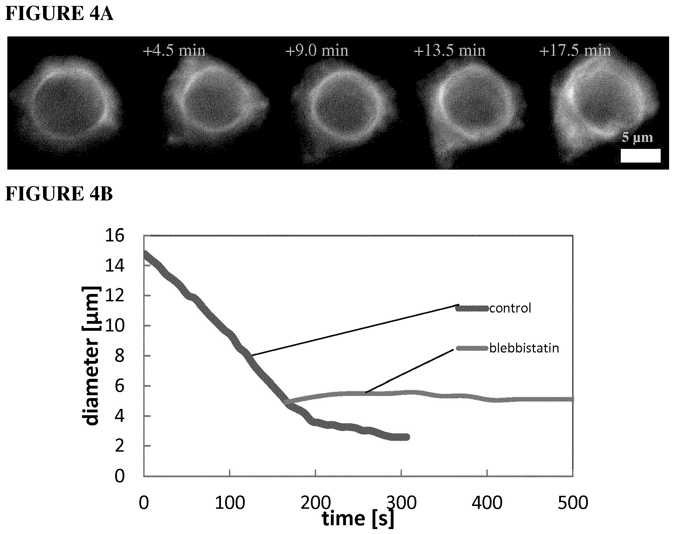

Blebbistatin is a molecule which inhibits myosin activity (Straight AF et al., Science. 2003, 299:1743-1747). The inventors observed that, at a concentration of 100µM, the closure was slowed down and failed (see Fig. 4).

For both drugs, the time of action was the same in egg cups and on glass coverslips. This demonstrates that the invention is suitable to observe quantitative and qualitative changes in cell division.

### Variations of well dimensions and consequences

At the onset of cytokinesis, cells round up. We measured the average diameter of the cells in this stage and found a value of 22.5 µm ± 0.5µm. Before reaching the division step, cells become spherical, then they elongate and due to the constriction in the center, they have a dumbbell shape. At the stage of maximal elongation, the long axis has a length of 31 µm ± 5 µm (see Fig. 7 A).

We probed systematically cavity sizes between 17.5µm and 27µm. This range covers the sizes of the non mitotic cells up to the sizes of elongating cells. We found that for small cavity sizes, cells were expulsed from the cavities before division (17.5 µm, 20 µm) (Fig. 7 C). For cavity sizes between 22µm and 25µm, we obtained a good filling percentage of the egg cup array, and perfectly oriented cytokinetic rings were observed. For increasing diameters of cavities, the plane of the cytokinetic ring was more and more tilted compared to the plane of observation (27 µm) (Fig. 7 B). For optimal results, we performed the experiments with cavity sizes of 25µm.

### Materials and Methods

### Microfabrication of the egg cups

A photolithography mask with black disks of the size of the later egg cup diameters was used (Selba S.A.). The diameter was varied between 17.5 µm and 27 µm. The negative photoresist SU-8 (Microchem) was spin coated for 30 s at 2100 rpm on a silicon wafer to obtain a layer of 40 µm. The layer was prebaked for 2 min on 65 °C and for 5 min on 95 °C. It was exposed for 51.5 s with a UV lamp (exposure energy 310 mJ/cm²). It followed a post exposure back of 1 min on 65 °C and for 3 min on 95 °C. The soluble parts of the SU-8 layer were washed away with SU-8 developer (Microchem).

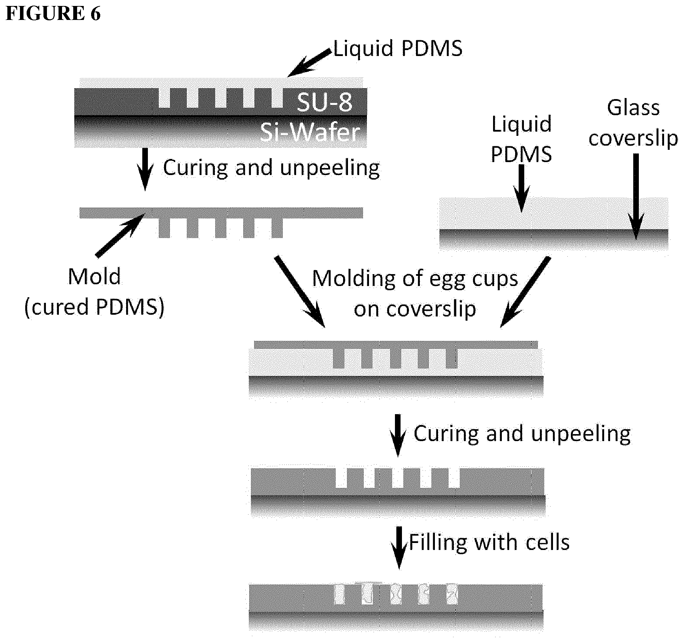

The egg cup fabrication is shown in Figure 6. Polydimethylsiloxane (PDMS) was mixed with the curing agent in a ratio of 1:10 (Sylgard 184). The air inclusions were removed by centrifuging the mixture for 5 min at 4000 rpm. The PDMS was poured on the silicon wafer with the SU-8 pattern and cured for at least 4 h at 65 °C. The cured PDMS could be easily unpeeled from the SU-8 and was used in the following as mold for the egg cups. The surface of the side which was on top of the SU-8 layer formed pillars. The molds were exposed to nitrogen plasma for 30 s and then incubated with chlorotrimethylsilane (Sigma Aldrich) vapor for 7 min.

Glass coverslips (# 0, diameter 25 mm, Fisherbrand) were exposed to nitrogen plasma for 30 s and spin coated with liquid PDMS at 1500 rpm for 30 s. The ratio curing agent:prepolymer corresponded to 1:10. The molds, which were prepared as described, were gently put on the thin PDMS layer with the pillar side on top of the PDMS. Air inclusions between the mold and the liquid PDMS left the interspace within one hour. After this the samples were cured for at least 4 h at 65 °C.

The mold could be carefully unpeeled from the PDMS layer on the coverslip. Accordingly, the wells or egg cups had a diameter varied between 17.5 µm and 27 µm and a depth of 40 µm.

### Cell lines

For the experiments, a human HeLa cell line was used. The cells were stably transfected with a plasmid coding for actin monomers tagged with GFP. Cells were cultured in a growth medium containing DMEM with 10 % FCS and 2 mM of L-Glutamine. After replating cells, Geneticin (0.5 mg/ml) was added to the medium.

During the experiments, cells were cultured in L-15 with 10% FCS and 2 mM of L-Glutamine.

### Preparation of the experiments

Synchronized cells were obtained by mitotic shake-off. To have a sufficient number of dividing cells, cells were cultured in flasks of 75 cm² surface. The flasks were tapped on a solid surface to detach loosely attached cells, which rounded up before undergoing division. Alternatively, cells were synchronized with classical protocols such as the thymidine block, mitotic block (both Whitfield ML, Mol. Cell. Biol. 2000, 20(12): 4188-4198) or monastrol incubation (Straight AF et al., Science. 2003, 299:1743-1747). They showed the same behavior as cells which were shook off.

The coverslips with the egg cups were exposed to nitrogen plasma for 30 s and then incubated with a solution of fibronectin (20 µg/ml in phosphate buffered saline (PBS), Sigma Aldrich) for 1 h. The sample could be stored in PBS for some hours. A cylindrically shaped plastic piece was placed in a 50 ml tube. The tube was filled with 6.5 ml of growth medium. The egg cup coverslip was horizontally placed in a tube, supported by the plastic cylinder. The suspension of synchronized cells was added on top of the coverslip. The tube was centrifuged for 5 min at 4000 rpm. The coverslip was carefully removed from the tube and installed in a home-made metallic holder. L-15 medium with 10 % FCS and 2 mM L-Glutamine was immediately added. During the experiment the holder was closed by a plastic cover to prevent evaporation.

In the case of fixed samples, coverslips were treated like classical samples. After filling of the egg cups with cells, they were fixed by incubation with paraformaldehyde (Sigma Aldrich) for 17 min. To stain the cells, the fixed samples were incubated with 0.5 % triton solution (Sigma Aldrich). After washing with Phosphate Buffer Saline (PBS, Gibco), cells were incubated for 45 min paxillin- antibody (Transduction Laboratories , Cat.No. 610051) and phosphotyrosine-antibody (Transduction Laboratories, Cat.No. 610009), respectively. After another washing with PBS, cells were incubated for 45 min with DAPI and a secondary antibody (Anti-Mouse, Jackson Immunoresearch, Cat.No. 115-165-145). The samples were stored in PBS at 4°C.

Cells observed on glass coverslips were not synchronized. The culture was plated one to three days before the experiment on the coverslips. They were observed in the same medium (L-15, 10% FCS, 2 mM L-Glutamine).

### Drugs

The drugs were added immediately before the experiment. The inventors used a concentration of 1.5 µM latrunculin A (Sigma Aldrich) (Delanoë-Ayari H.et al., Phys. Rev. Lett. 2004, 93(10): 108102) and 100 µM (-)-blebbistatin (Sigma Aldrich) (Straight AF et al., Science. 2003, 299:1743-1747).

### Microscope

Cells were observed with an inverted microscope: Nikon Eclipse Ti microscope (Nikon) equipped with a CCD camera coolSNAP HQ² (Photometrics), a Lambda DG-4 lamp (Sutter Instrument Company) and a temperature control system from Life Imaging Services (heater: the cube 2, plexiglass cage: the box). The objective was a Plan Apo 60x objective (oil, 1.40 NA, Nikon). The images were captured and processed with the NIS Elements software (v3.10, SP3, Nikon).

## Claims

1. A method for observing eukaryotic cells without cell wall, comprising
- Providing a device comprising a plurality of wells suitable for containing only one single eukaryotic cell, wherein the width of the wells is about the diameter of the cells in suspension at a resting phase, more or less 5 or 10 %, the dimensions of the wells being suitable for constraining the cells during mitosis into an oblong shape with a long axis parallel to the depth of the wells, wherein the cells contained in said wells have the closure plane of their cytokinetic ring parallel to the observation plan and wherein the device is not used with a top covering the upper surface of the wells and being coated with molecules promoting cell attachment; and
- Observing the cells placed into the wells.

2. The method of claim 1, wherein the ratio between the wells width and the wells depth is less than 0.8.

3. The method according to claim 1 or 2, wherein the depth of the wells is less than two diameters of the cells in suspension, in particular at a resting phase.

4. The method of any one of claims 1-3, wherein the method further comprises a previous step of selecting the suitable device for observing the cells of interest, in particular based on the cells size.

5. The method according to any one of claims 1-4, wherein the step of observing cells comprises one or several of the following steps, optionally for a period of time,:
- determining the level of expression of proteins; and/or,
- determining the level of activity of proteins, and/or
- determining the localization or the interaction of proteins; and/or,
- observing the structure, localization, shape and/or integrity of organelles, cytoskeleton, DNA, or cytokinetic ring; and/or
- observing cell apoptosis; and/or
- observing the cytokinetic ring, in particular its closure; and/or
- observing the effect of molecules, antibodies, drugs, or siRNA on the level of expression of proteins, the level of activity of proteins, the localization or interaction of proteins, the structure, localization, shape and/or integrity of organelles, cytoskeleton, DNA, or cytokinetic ring, the closure of the cytokinetic ring.

6. The method according to any one of claims 1-5, wherein the width of the wells is between about 12 to 30 µm, preferably about 20 µm.

7. The method according to any one of claims 1-6, wherein the interior surface of the wells is coated with molecules that promote cell attachment, preferably fibronectin.

8. The method according to any one of claims 1-7, wherein the device comprises a microfabricated substrate, preferably made of poly(dimethylsiloxane) (PDMS), preferably supported by a plate, more preferably of glass, still more preferably a glass coverslip.

9. The method according to any one of claims 1-8, wherein the cells are superior eukaryotic cells, in particular mammalian cells.

10. The method according to any one of claims 1-9, wherein the cells have been placed into the wells by a centrifugation step.

11. Use of a method according to any one of claims 1-10 for screening or identifying a molecule of interest, in particular a molecule able to modulate the cell division.

12. Use of a method according to any one of claims 1-10 for *in vitro* diagnosing a disease, in particular a proliferative disease such as tumor or a cancer.

13. Use of a method according to any one of claims 1-10 for assessing the responsiveness or the toxicity to a drug.

14. A method for screening or identifying a molecule able to modulate the cell division, in particular a molecule able to modulate the closure of the cytokinetic ring, comprising
- performing the method of any one of claims 1-10 with cells in presence and in absence of a test molecule;
- assessing the closure of the cytokinetic ring of the cells in presence and in absence of the test molecule;
- comparing the closure of the cytokinetic ring of the cells in presence and in absence of the test molecule; and,
- selecting the test molecule for which the closure is significantly different in presence and in absence of the test molecule, thereby identifying a test molecule able to modulate the cell division.

15. A method for *in vitro* diagnosis of a proliferative disorder in a subject comprising:
- performing the method of any one of claims 1-10 with cells of a sample from the subject;
- assessing the closure of the cytokinetic ring of the cells; and,
- comparing the closure of the cytokinetic ring of the cells to the closure of the ring of reference cells.

## Patentansprüche

1. Verfahren zur Beobachtung eukaryotischer Zellen ohne Zellwand, umfassend
- Bereitstellen einer Vorrichtung, umfassend eine Vielzahl von Vertiefungen, die geeignet sind, um nur eine einzige eukaryotische Zelle zu enthalten, wobei die Breite der Vertiefungen ungefähr der Durchmesser der Zellen in Suspension in einer Ruhephase ist, mehr oder weniger 5 oder 10 %, wobei die Abmessungen der Vertiefungen geeignet sind, um die Zellen während der Mitose in eine längliche Form zu zwingen, mit der langen Achse parallel zur Tiefe der Vertiefungen, wobei die Zellen, die in den Vertiefungen enthalten sind, die Verschlussebene ihres zytokinetischen Rings parallel zur Beobachtungsebene aufweisen, und wobei die Vorrichtung nicht mit einer Oberseite verwendet wird, die die obere Fläche der Vertiefungen abdeckt und mit Molekülen bedeckt ist, die die Zellhaftung fördern; und
- Beobachten der Zellen, die in die Vertiefungen gegeben sind.

2. Verfahren nach Anspruch 1, wobei das Verhältnis zwischen der Vertiefungsbreite und der Vertiefungstiefe weniger als 0,8 ist.

3. Verfahren nach Anspruch 1 oder 2, wobei die Tiefe der Vertiefungen weniger als zwei Durchmesser der Zellen in Suspension ist, insbesondere in einer Ruhephase.

4. Verfahren nach einem der Ansprüche 1 - 3, wobei das Verfahren weiter einen vorherigen Schritt des Auswählens der geeigneten Vorrichtung umfasst, um die betreffenden Zellen zu beobachten, insbesondere basierend auf der Zellengröße.

5. Verfahren nach einem der Ansprüche 1 - 4, wobei der Schritt des Beobachtens von Zellen einen oder mehrere der folgenden Schritte umfasst, optional für einen Zeitraum:
- Bestimmen des Expressionsniveaus von Proteinen; und/oder
- Bestimmen des Aktivitätsniveaus von Proteinen, und/oder
- Bestimmen der Lokalisierung oder der Interaktion von Proteinen; und/oder
- Beobachten der Struktur, Lokalisierung, Form und/oder Integrität von Organellen, Zytoskelett, DNA oder zytokinetischem Ring; und/oder
- Beobachten der Zellapoptose; und/oder
- Beobachten des zytokinetischen Rings, insbesondere seines Verschlusses; und/oder
- Beobachten der Wirkung von Molekülen, Antikörpern, Arzneimitteln oder siRNA auf das Expressionsniveau von Proteinen, das Aktivitätsniveau von Proteinen, die Lokalisierung oder Interaktion von Proteinen, die Struktur, Lokalisierung, Form und/oder Integrität von Organellen, Zytoskelett, DNA oder zytokinetischem Ring, den Verschluss des zytokinetischen Rings.

6. Verfahren nach einem der Ansprüche 1 - 5, wobei die Breite der Vertiefungen zwischen ungefähr 12 bis 30 µm, vorzugsweise ungefähr 20 µm liegt.

7. Verfahren nach einem der Ansprüche 1 - 6, wobei die innere Oberfläche der Vertiefungen mit Molekülen beschichtet ist, die die Zellhaftung fördern, vorzugsweise Fibronectin.

8. Verfahren nach einem der Ansprüche 1 - 7, wobei die Vorrichtung ein mikrofabriziertes Substrat umfasst, vorzugsweise hergestellt aus Poly(dimethylsiloxan) (PDMS), vorzugsweise getragen von einer Platte, insbesondere von Glas, noch bevorzugter einem Deckglas.

9. Verfahren nach einem der Ansprüche 1 - 8, wobei die Zellen höhere eukaryotische Zellen sind, insbesondere Säugetierzellen.

10. Verfahren nach einem der Ansprüche 1 - 9, wobei die Zellen durch einen Schritt des Zentrifugierens in die Vertiefungen gegeben wurden.

11. Verwendung eines Verfahrens nach einem der Ansprüche 1 - 10 zum Screening oder Identifizieren eines bestimmten Moleküls, insbesondere eines Moleküls, das dazu in der Lage ist, die Zellteilung zu modulieren.

12. Verwendung eines Verfahrens nach einem der Ansprüche 1 - 10 zur *in vitro-*Diagnose einer Erkrankung, insbesondere einer proliferativen Erkrankung wie z. B. einem Tumor oder einem Krebs.

13. Verwendung eines Verfahrens nach einem der Ansprüche 1 - 10 zur Bewertung der Reaktionsfähigkeit oder der Toxizität für ein Arzneimittel.

14. Verfahren zum Screening oder Identifizieren eines Moleküls, das dazu in der Lage ist, die Zellteilung zu modulieren, insbesondere eines Moleküls, das dazu in der Lage ist, den Verschluss des zytokinetischen Rings zu modulieren, umfassend
- Durchführen des Verfahrens nach einem der Ansprüche 1 - 10 mit Zellen in Anwesenheit oder in Abwesenheit eines Testmoleküls;
- Bewerten des Verschlusses des zytokinetischen Rings der Zellen in Anwesenheit oder in Abwesenheit des Testmoleküls;
- Vergleichen des Verschlusses des zytokinetischen Rings der Zellen in Anwesenheit oder in Abwesenheit des Testmoleküls; und
- Auswählen des Testmoleküls, für das der Verschluss signifikant verschieden in Anwesenheit oder in Abwesenheit des Testmoleküls ist, wodurch ein Testmolekül identifiziert wird, das dazu in der Lage ist, die Zellteilung zu modulieren.

15. Verfahren zur *in vitro*-Diagnose einer proliferativen Störung in einem Subjekt, umfassend:
- Durchführen des Verfahrens nach einem der Ansprüche 1 - 10 mit Zellen einer Probe vom Subjekt;
- Bewerten des Verschlusses des zytokinetischen Rings der Zellen; und
- Vergleichen des Verschlusses des zytokinetischen Rings der Zellen mit dem Verschluss des Rings von Referenzzellen.

## Revendications

1. Méthode d'observation de cellules eucaryotes sans paroi cellulaire, comprenant
- la fourniture d'un dispositif comprenant une pluralité de puits adaptés pour contenir uniquement une seule cellule eucaryote, dans laquelle la largeur des puits est environ le diamètre des cellules en suspension à une phase de repos, plus ou moins 5 ou 10 %, les dimensions des puits étant adaptées pour contraindre les cellules pendant une mitose en une forme oblongue avec un axe long parallèle à la profondeur des puits, dans laquelle les cellules contenues dans lesdits puits ont le plan de fermeture de leur anneau de cytocinèse parallèle au plan d'observation et dans laquelle le dispositif n'est pas utilisé avec une partie haute couvrant la surface supérieure des puits et revêtue de molécules favorisant la fixation de cellules ; et
- l'observation des cellules placées dans les puits.

2. Méthode selon la revendication 1, dans laquelle le rapport entre la largeur des puits et la profondeur des puits est inférieur à 0,8.

3. Méthode selon la revendication 1 ou 2, dans laquelle la profondeur des puits est inférieure à deux diamètres des cellules en suspension, en particulier à une phase de repos.

4. Méthode selon l'une quelconque des revendications 1 à 3, dans laquelle la méthode comprend en outre une étape préalable de sélection du dispositif adapté pour observer les cellules d'intérêt, en particulier d'après la taille des cellules.

5. Méthode selon l'une quelconque des revendications 1 à 4, dans laquelle l'étape d'observation de cellules comprend une ou plusieurs des étapes suivantes, facultativement pendant une période de temps :
- la détermination du taux d'expression de protéines ; et/ou,
- la détermination du taux d'activité de protéines, et/ou
- la détermination de la localisation ou de l'interaction de protéines ; et/ou,
- l'observation de la structure, de la localisation, de la forme et/ou de l'intégrité d'organelles, du cytosquelette, de l'ADN, ou d'un anneau de cytocinèse ; et/ou
- l'observation d'une apoptose cellulaire ; et/ou
- l'observation de l'anneau de cytocinèse, en particulier sa fermeture ; et/ou
- l'observation de l'effet de molécules, d'anticorps, de médicaments, ou d'ARNsi sur le taux d'expression de protéines, le taux d'activité de protéines, la localisation ou l'interaction de protéines, la structure, la localisation, la forme et/ou l'intégrité d'organelles, du cytosquelette, de l'ADN, ou d'un anneau de cytocinèse, la fermeture de l'anneau de cytocinèse.

6. Méthode selon l'une quelconque des revendications 1 à 5, dans laquelle la largeur des puits est entre environ 12 et 30 µm, de préférence d'environ 20 µm.

7. Méthode selon l'une quelconque des revendications 1 à 6, dans laquelle la surface intérieure des puits est revêtue de molécules qui favorisent la fixation de cellules, de préférence la fibronectine.

8. Méthode selon l'une quelconque des revendications 1 à 7, dans laquelle le dispositif comprend un substrat microfabriqué, de préférence réalisé en poly(diméthylsiloxane) (PDMS), de préférence supporté par une plaque, de manière davantage préférée en verre, de manière encore davantage préférée une lamelle couvre-objet en verre.

9. Méthode selon l'une quelconque des revendications 1 à 8, dans laquelle les cellules sont des cellules eucaryotes supérieures, en particulier des cellules de mammifère.

10. Méthode selon l'une quelconque des revendications 1 à 9, dans laquelle les cellules ont été placées dans les puits par une étape de centrifugation.

11. Utilisation d'une méthode selon l'une quelconque des revendications 1 à 10 pour le dépistage ou l'identification d'une molécule d'intérêt, en particulier d'une molécule capable de moduler la division cellulaire.

12. Utilisation d'une méthode selon l'une quelconque des revendications 1 à 10 pour le diagnostic *in vitro* d'une maladie, en particulier une maladie proliférative telle qu'une tumeur ou un cancer.

13. Utilisation d'une méthode selon l'une quelconque des revendications 1 à 10 pour évaluer la réactivité ou la toxicité à un médicament.

14. Méthode de dépistage ou d'identification d'une molécule capable de moduler la division cellulaire, en particulier d'une molécule capable de moduler la fermeture de l'anneau de cytocinèse, comprenant
- la réalisation de la méthode de l'une quelconque des revendications 1 à 10 avec des cellules en présence et en l'absence d'une molécule d'essai ;
- l'évaluation de la fermeture de l'anneau de cytocinèse des cellules en présence et en l'absence de la molécule d'essai ;
- la comparaison de la fermeture de l'anneau de cytocinèse des cellules en présence et en l'absence de la molécule d'essai ; et,
- la sélection de la molécule d'essai pour laquelle la fermeture est significativement différente en présence et en l'absence de la molécule d'essai, identifiant ainsi une molécule d'essai capable de moduler la division cellulaire.

15. Méthode de diagnostic *in vitro* d'un trouble prolifératif chez un sujet comprenant :
- la réalisation de la méthode de l'une quelconque des revendications 1 à 10 avec des cellules d'un échantillon provenant du sujet ;
- l'évaluation de la fermeture de l'anneau de cytocinèse des cellules ; et,
- la comparaison de la fermeture de l'anneau de cytocinèse des cellules à la fermeture de l'anneau de cellules de référence.
